# EUROPÄISCHE PATENTSCHRIFT

(11) **EP 3 458 125 B1**
(45) Veröffentlichungstag und Bekanntmachung des Hinweises auf die Patenterteilung: **06.10.2021**
(21) Anmeldenummer: 17724794.7
(22) Anmeldetag: 16.05.2017
(51) Int. Cl.: A61M 1/14, A61M 1/30, A61M 1/34

(54) **MEDIZINISCHE KONDENSATFALLE ZUR MEDIZINISCHEN VERWENDUNG, VERFAHREN ZUR ENTFEUCHTUNG, BLUTBEHANDLUNGSVORRICHTUNG, BLUTBEHANDLUNGSEINRICHTUNG**
MEDICAL CONDENSATE TRAP FOR MEDICAL USE, METHOD FOR DEHUMIDIFYING, BLOOD TREATMENT DEVICE, BLOOD TREATMENT APPARATUS
PIÈGE À CONDENSAT MÉDICAL POUR UNE UTILISATION MÉDICALE, PROCÉDÉ DE DÉSHUMIDIFICATION, DISPOSITIF DE TRAITEMENT DU SANG ET SYSTÈME DE TRAITEMENT DU SANG

(30) Priorität: 20.05.2016 DE 102016109340
(43) Veröffentlichungstag der Anmeldung: 27.03.2019
(73) Patentinhaber: Fresenius Medical Care Deutschland GmbH, 61352 Bad Homburg (DE)
(72) Erfinder: MÜLLER, Ralf, 61350 Bad Homburg (DE); NOACK, Joachim, 97616 Bad Neustadt (DE)
(74) Vertreter: Bobbert & Partner Patentanwälte PartmbB
(86) Internationale Anmeldenummer: PCT/EP2017/061739
(87) Internationale Veröffentlichungsnummer: WO 2017/198670

(56) Entgegenhaltungen:
- WO-A2-90/04425
- US-A- 5 863 421
- US-A1- 2011 077 605
- US-A1- 2014 112 828

## Beschreibung

Die vorliegende Erfindung betrifft eine medizinische Kondensatfalle gemäß Anspruch 1. Sie betrifft zudem ein Verfahren gemäß Anspruch 7, eine Blutbehandlungsvorrichtung gemäß Anspruch 10 und eine Blutbehandlungseinrichtung gemäß Anspruch 14.

Aus dem Stand der Technik sind Blutbehandlungsvorrichtungen für die extrakorporale Blutbehandlung von Blut bekannt. Dabei wird Blut durch Disposables wie Blutschläuche oder Blutkassetten (im Folgenden kurz: Behandlungseinrichtungen) geführt, bevor es in das Gefäßsystem des Patienten zurückgeführt wird. Bei der Blutbehandlung kann es zu unerwünschter Kondensierung von Luftfeuchtigkeit an den verwendeten Disposables oder an Komponenten der eingesetzten Blutbehandlungsvorrichtung kommen.

Aus der US 2011/077605 A1 ist ein Pumpensystem für die Unterdruck-Wundbehandlung bekannt.

In der WO 90/04425 A2 sind eine Vorrichtung zur Gasprobenentnahme und eine Wasserfalle offenbart.

Eine Hämodialysevorrichtung mit automatischem Priming mittels induzierter Druckimpulse ist aus der US 5863421 A bekannt.

Eine Hämodialysevorrichtung mit einer Abfluss-Kassette ist in der US 2014/112828 A1 offenbart.

Eine Aufgabe der vorliegenden Erfindung ist es, Vorrichtungen und Verfahren zur Entfeuchtung bei der Blutbehandlung vorzuschlagen.

Die erfindungsgemäße Aufgabe kann durch eine medizinische Kondensatfalle mit den Merkmalen des Anspruchs 1 gelöst werden. Sie kann ferner gelöst werden mittels des Verfahrens mit den Merkmalen des Anspruchs 7, mittels der Blutbehandlungsvorrichtung mit den Merkmalen des Anspruchs 10 und mittels einer Blutbehandlungseinrichtung mit den Merkmalen des Anspruchs 14.

Erfindungsgemäß wird somit eine medizinische Kondensatfalle zur Verwendung bei der externen Blutbehandlung mittels einer Blutbehandlungsvorrichtung vorgeschlagen. Die Kondensatfalle weist wenigstens ein Inneres zur Aufnahme eines Gases auf. Sie weist wenigstens einen ersten Anschluss auf, der in Fluidkommunikation mit dem Inneren steht, und welcher zur Verbindung des Inneren mit beispielsweise einem Gasausgang der Blutbehandlungsvorrichtung dienen kann.

Die Kondensatfalle weist ferner wenigstens einen zweiten Anschluss zur Verbindung des Inneren in Fluidkommunikation auf, beispielsweise zur Verbindung mit einem Gaseingang der Blutbehandlungsvorrichtung.

Schließlich weist die Kondensatfalle wenigstens einen dritten Anschluss zur Verbindung des Inneren in Fluidkommunikation auf, beispielsweise zur Verbindung mit einem Luftport oder pneumatischen Anschluss einer Blutbehandlungseinrichtung, etwa einem Blutschlauchsatz oder einer Blutkassette.

Die Verbindung kann dabei jeweils z. B. direkt (ohne Einsatz eines Verbindungsschlauchs) oder indirekt (also z. B. mittels Verbindungsschlauchs) erfolgen.

Das erfindungsgemäße Verfahren dient dem Entfeuchten wenigstens eines Abschnitts einer Blutbehandlungseinrichtung und/oder eines Abschnitts einer Blutbehandlungsvorrichtung, etwa einer Single-Needle-Pneumatik-Einheit, eines Kompressors oder Abschnitten oder Komponenten jeweils hiervon wie z. B. Pneumatikschläuchen oder Kompressorabschnitten.

Das Verfahren umfasst das Bereitstellen einer Blutbehandlungsvorrichtung, einer erfindungsgemäßen Kondensatfalle und einer Blutbehandlungseinrichtung.

Die Blutbehandlungsvorrichtung weist einen Gasausgang und einen getrennt hiervon ausgestalteten Gaseingang auf, die beispielsweise als Ports ausgestaltet sind. Ferner weist die Blutbehandlungsvorrichtung eine Blutpumpe und einen Kompressor zum Erzeugen von Druckluft auf. Der Kompressor steht in Fluidverbindung mit dem Gasausgang.

Die Blutbehandlungseinrichtung weist eine Blutleitung oder Blutkammer, einen Luftport und eine Hydrophobmembran (auch als hydrophober Filter bezeichnet) auf. Der Luftport ist in Fluidkommunikation mit der Hydrophobmembran angeordnet. Die dem Luftport zugewandte Seite der Hydrophobmembran wird hierin als seine luftport-zugewandte Seite bezeichnet.

Der Luftport der Blutbehandlungseinrichtung ist mit dem Gasausgang der Blutbehandlungsvorrichtung verbunden. Die Blutleitung oder die Blutkammer sind mit der Blutpumpe verbunden. Die Blutleitung oder die Blutkammer stehen insbesondere ebenfalls in Fluidkommunikation mit dem Luftport

Das Verfahren umfasst ein wenigstens einmaliges Durchführen eines Entlüftungsvorgangs, welches die folgenden Schritte umfasst: Aufbauen eines vorbestimmten Drucks oder Mindestdrucks innerhalb der Blutbehandlungseinrichtung auf der luftport-abgewandten Seite der Hydrophobmembran in Richtung des ersten Anschlusses mittels der Blutpumpe und/oder mittels des Kompressors; und Abbau des Drucks durch Öffnen eines Ventils in Richtung der Hydrophobmembran; und optional: Fördern mit dem Kompressor bei geöffnetem Ventil zwischen Kondensatauffang und Kompressoreingang.

Die Erfindung betrifft ferner eine Blutbehandlungsvorrichtung, welche wenigstens eine Einrichtung zum Abscheiden von Kondensat aufweist oder hiermit verbunden ist.

Die erfindungsgemäße Blutbehandlungseinrichtung ist ein Blutschlauchsatz, oder ein Teil hiervon, und/oder eine Blutkassette. Sie umfasst eine erfindungsgemäße Kondensatfalle oder ist hiermit verbunden.

Bei allen Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Erfindungsgemäße Ausführungsformen können eines oder mehrere der vorstehend oder im Folgenden genannten Merkmale aufweisen. Erfindungsgemäße Ausführungsformen sind ferner Gegenstand der Unteransprüche.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Die Angaben "oben" und "unten" sind hierin bei Zweifel des Fachmanns als absolute oder relative Raumangaben zu verstehen, welche sich auf die Ausrichtung des betreffenden Bauteils während seines üblichen Gebrauchs und/oder die Darstellung in den hier beigefügten Figuren beziehen.

Die vorliegende Erfindung betrifft jede beliebige Kombination von hierin genannten Merkmalen, sofern eine konkrete Kombination nicht für den Fachmann erkennbar technisch unmöglich ist.

In manchen beispielhaften, erfindungsgemäßen Ausführungsformen weist die Kondensatfalle genau drei Anschlüsse auf, nämlich den ersten, zweiten und dritten.

-Erfindungsgemäß ist der dritte Anschluss in einer Gebrauchsstellung der Kondensatfalle höher angeordnet als der erste Anschluss und als der zweite Anschluss.

Erfindungsgemäß ist der erste Anschluss in einer Gebrauchsstellung der Kondensatfalle höher angeordnet als der zweite Anschluss.

In manchen beispielhaften, erfindungsgemäßen Ausführungsformen ist der dritte Anschluss einem ersten Endbereich des Inneren oder einem ersten Endbereich der Kondensatfalle zugeordnet. Der zweite Anschluss ist einem zweiten, dem ersten Endbereich gegenüberliegenden Endbereich des Inneren oder der Kondensatfalle zugeordnet. Der erste Anschluss ist zwischen dem zweiten Anschluss und dem dritten Anschluss angeordnet.

In manchen beispielhaften, erfindungsgemäßen Ausführungsformen ist das Innere, das sich in einer Längsrichtung und in einer Querrichtung erstreckt, mittels eines Gehäuses vom Äußeren der Kondensatfalle getrennt oder diesem gegenüber abgedichtet.

Das Gehäuse weist wenigstens eine erste Öffnung für einen Gasaustausch mit dem Inneren über den ersten Anschluss, wenigstens eine zweite Öffnung für einen Gasaustausch mit dem Inneren über den zweiten Anschluss und wenigstens eine dritte Öffnung für einen Gasaustausch mit dem Inneren über den dritten Anschluss auf.

In manchen beispielhaften, erfindungsgemäßen Ausführungsformen entspricht der Abstand zwischen der ersten Öffnung und der dritten Öffnung mindestens der Erstreckung in Querrichtung des Inneren. Alternativ ist er größer als diese Erstreckung. Die erste und die dritte Öffnung können erfindungsgemäß vertauscht sein, was noch immer unter die Erfindung fällt. Vorzugsweise liegt die Öffnung über dem Pegel.

In manchen beispielhaften, erfindungsgemäßen Ausführungsformen entspricht der Abstand zwischen der ersten Öffnung und der zweiten Öffnung mindestens der Erstreckung in Querrichtung. Alternativ ist er größer als diese Erstreckung.

In manchen beispielhaften, erfindungsgemäßen Ausführungsformen sind der Durchmesser und/oder eine Fläche der ersten Öffnung, der zweiten Öffnung und/oder der dritten Öffnung oder die Innendurchmesser der mit den Anschlüssen verbundenen Schläuche oder Verbindungsleitungen kleiner als die Erstreckung des Inneren in Querrichtung. Ein hiermit erzielbarer Vorteil kann darin bestehen, dass ein Durchschieben von Kondensattropfen in Richtung Luftport oder pneumatischem Anschluss durch die so erzielte Querschnittsaufweitung zum Inneren hin erschwert oder verhindert werden kann.

In manchen erfindungsgemäßen, beispielhaften Ausführungsformen weist die Blutbehandlungsvorrichtung einen Single-Needle-Tank auf. Die Blutbehandlungseinrichtung ist eine Blutkassette. Die Blutleitung oder Blutkammer ist oder umfasst eine Single-Needle-Kammer. Die Hydrophobmembran ist in einer Fluidverbindung zwischen dem Luftport und der Single-Needle-Kammer angeordnet. Der vorbestimmte Druck oder Mindestdruck wird in der Single-Needle-Kammer aufgebaut. Der Druck wird in Richtung eines Single-Needle-Tanks der Blutbehandlungsvorrichtung oder in die Umgebung abgebaut. Der Abschnitt der Blutbehandlungsvorrichtung, in welchen mittels Kompressors bei geöffnetem Ventil gefördert wird, ist ein Abschnitt eine Single-Needle-Pneumatik-Einheit.

In manchen erfindungsgemäßen, beispielhaften Ausführungsformen umfasst das Verfahren weiter, dass der Entlüftungsvorgang ausgeführt oder gestartet wird, wenn eine vorbestimmte Bedingung erfüllt ist. Diese kann der Ablauf einer vorbestimmten Zeitdauer seit der zuletzt durchgeführten Entlüftung sein; das Erreichen/Unterschreiten einer vorbestimmten Temperatur, insbesondere mittels Sensors im Bereich des Luftports oder des pneumatischen Anschlusses; das Erreichen/Unterschreiten eines vorbestimmten Flussverhältnisses, gemessen mit einem Flusssensor der Blutbehandlungsvorrichtung, welcher an geeigneter Stelle vorgesehen sein kann; das Detektieren einer vorbestimmten Wellenlänge bei Transmissions- oder Streulichtmessungen am Gaseingang.

In manchen erfindungsgemäßen, beispielhaften Ausführungsformen ist die Einrichtung zum Abscheiden von Kondensat eine erfindungsgemäße Kondensatfalle oder umfasst eine solche.

Erfindungsgemäß weist die Blutbehandlungsvorrichtung einen Gasausgang und einen getrennt hiervon ausgestalteten Gaseingang auf.

Erfindungsgemäß weist die Blutbehandlungsvorrichtung einen Kompressor oder eine andere Einrichtung zum Erzeugen von Druckluft und einen Kondensatauffang auf. Der Kompressor ist mit dem Gasausgang verbunden. Der Kondensatauffang ist mit dem Gaseingang verbunden.

In manchen erfindungsgemäßen, beispielhaften Ausführungsformen ist die Blutbehandlungsvorrichtung verbunden mit einer Blutbehandlungseinrichtung, welche als Blutschlauchsatz (oder Teil hiervon) und/oder als Blutkassette ausgestaltet ist, welche(r) eine Hydrophobmembran aufweist. Die Kondensatfalle ist dabei in einer Fluidverbindung zwischen der Hydrophobmembran und dem Gasausgang und/oder Gaseingang angeordnet.

In manchen erfindungsgemäßen, beispielhaften Ausführungsformen ist die Einrichtung zum Abscheiden von Kondensat ein Feuchtetauscher oder umfasst einen solchen. Ein Feuchtetauscher ist im Sinne der Erfindung vorzugsweise eine Vorrichtung, die zumindest in Teilen eine hohe Permeabilität für Wasser(dampf) aufweist und zugleich gasdicht ist oder zumindest eine hohe Gasdichtheit aufweist. Mit Hilfe eines Feuchtetauschers kann in einigen Ausführungsformen Feuchtigkeit aus einem Gas (insbesondere Luft) der Blutbehandlungsvorrichtung, oder aus einem Gas (insbesondere Luft), welches durch diese oder einen angeschlossenen, extrakorporalen Blutkreislauf geführt wird, entfernt werden. Der Feuchtetauscher ist in einigen Ausführungsformen Teil eines Disposables der Blutbehandlungsvorrichtung. In einigen Ausführungsformen umfasst der Feuchtetauscher Nafion.

In manchen erfindungsgemäßen, beispielhaften Ausführungsformen ist die Einrichtung zum Abscheiden von Kondensat eine Feuchtefalle oder umfasst eine Feuchtefalle, welche mittels eines Kühlelements, etwa eines Peltierelements, Kondensat abscheidet und/oder bindet.

In manchen erfindungsgemäßen, beispielhaften Ausführungsformen ist die Blutbehandlungsvorrichtung ausgestaltet als Akut-Dialysevorrichtung, Dialysevorrichtung, Hämodialysevorrichtung, Hämofiltrationsvorrichtung, Apheresevorrichtung, Plasmabehandlungs- oder Austauschvorrichtung, TPE-(Therapeutic Plasma Exchange)-Vorrichtung, und/oder Kombinationen hiervon.

In manchen beispielhaften, erfindungsgemäßen Ausführungsformen weist die medizinische Behandlungsvorrichtung eine Steuer- oder Regelvorrichtung, auf, welche programmiert ist, um manchen oder alle Schritte des erfindungsgemäßen Verfahrens, insbesondere die Schritte des Entlüftungsvorgangs, auszuführen oder zu veranlassen.

In manchen beispielhaften, erfindungsgemäßen Ausführungsformen weist die Single-Needle-Pneumatik-Einheit den Kompressor, den Kondensatauffang und einen Single-Needle-Tank auf und/oder kann mit einer Anordnung gleichgesetzt werden, die die vorstehenden Bauteile umfasst.

In manchen beispielhaften, erfindungsgemäßen Ausführungsformen steht der Kompressor mittels Fluidleitung direkt mit dem Gasausgang der Blutbehandlungsvorrichtung in Fluidverbindung. Direkt kann bedeuten, dass allein eine Schlauchverbindung ohne weitere in den Fluidweg integrierte Komponenten vorgesehen ist.

In manchen beispielhaften, erfindungsgemäßen Ausführungsformen weist die Single-Needle-Pneumatik-Einheit einen Kondensatverdunster auf, welcher mittels einer Leitung insbesondere direkt, mit dem Gaseingang in Fluidverbindung steht.

Einige oder alle erfindungsgemäßen Ausführungsformen können einen oder mehrere der oben oder im Folgenden genannten Vorteile aufweisen.

Die erfindungsgemäße Kondensatfalle erlaubt es, Kondenswasser, welches z. B. im Luftport einer Blutbehandlungsvorrichtung steht, zu sammeln. Dieses kann anschließend abgeleitet werden.

Durch Verringern der Feuchtigkeit durch Absondern von Kondensat kann insbesondere bei Blutbehandlungsoptionen, bei welchen hydrophobe Filter oder Hydrophobmembrane in einem Gasströmungsweg verwendet werden, die Benetzung von Filter oder Membran verhindert werden. Mit Flüssigkeit benetzte hydrophobe Filter werden für Gas undurchlässig. Geschieht dies während der Blutbehandlung, so kann dies erfordern, die Blutbehandlung abzubrechen. Die vorliegende Erfindung kann dem vorteilhaft entgegenwirken.

Damit bietet die vorliegende Erfindung insbesondere für solche Blutbehandlungen einen Vorteil, bei welchen vergleichsweise große Mengen an feuchtigkeitsgesättigter Luft wechselweise in einen Behälter und wieder aus diesem heraus gefördert werden. Letzteres ist aus Single-Needle-Blutbehandlungen bekannt, bei welchen Luft aus einem Single-Needle-Kammer einer Blutkassette durch einen hydrophoben Filter hindurch in einen maschinenseitigen Druckspeicher (den Single-Needle-Tank) überführt und darin gespeichert wird, bis sie der Blutkassette durch den hydrophoben Filter hindurch wieder zugeführt wird. Auf dem Weg in den Druckspeicher wird die Luft über Pneumatikleitungen geführt, wobei sie sich abkühlt. Feuchtigkeit schlägt sich aufgrund von Temperaturunterschieden in Form von Tropfen an der Innenseite der Pneumatikleitungen nieder. Strömt die Luft im nächsten Zyklus aus dem Druckspeicher zurück in die Single-Needle-Kammer, so wird sie hierbei vom Kompressor optional mehr oder weniger stark komprimiert, was zu einer Feuchtigkeitsübersättigung führen kann. Kondensat legt sich schließlich auch am Ausgang des Kompressors ab. Einmal vorhandene Kondensattropfen dienen als Kondensationskeime und wachsen, bis sie die pneumatischen Leitungen im Querschnitt soweit einengen, dass sie vom Luftstrom mitgerissen werden können.

Da aus Gründen der Behandlungseffizienz in der venösen Phase in der Regel ein im Vergleich zur arteriellen Phase erhöhter Blutfluss verwendet wird, werden Kondensattropfen bevorzugt in Richtung hydrophober Filter/Hydrophobmembran der Blutkassette mitgerissen. Gelangt Kondensat auf die hydrophobe Membran, so wird diese für Luft unpassierbar, die Behandlung muss abgebrochen werden.

Kondensat kann erfindungsgemäß somit weitestgehend abgefangen werden, wodurch eine Benetzung des hydrophoben Filters vorteilhaft vermieden wird.

In einigen Ausführungsformen enthält die Kondensatfalle keinen Filter.

In einigen Ausführungsformen ist die Kondensatfalle als eine Kammer ausgeführt, d. h. die Kondensatfalle umfasst dann z. B. nicht zwei oder mehr Kammern, die z. B. durch eine Trennwand getrennt sind und/oder durch z. B. eine Öffnung verbunden sind.

In einigen Ausführungsformen umfasst die Kondensatfalle keinen Druckluftfilter, keine Drallscheibe und/oder keinen Sinterfilter.

Die vorliegende Erfindung kann mit geringen Kosten umgesetzt werden. Die erfindungsgemäße Kondensatfalle eignet sich, um nachgerüstet zu werden. Sie kann hygienisch vorteilhaft als Disposable oder Einwegartikel gefertigt sein.

Weitere Vorteile sind insbesondere zu den in den Figuren gezeigten, exemplarischen Ausführungsformen im Folgenden beschrieben.

Die vorliegende Erfindung wird im Folgenden anhand der beigefügten Zeichnungen, in welcher identische Bezugszeichen gleiche oder ähnliche Bauteile bezeichnen, exemplarisch erläutert. In den zum Teil stark vereinfachten Figuren gilt:
- Fig. 1: zeigt eine Kondensatfalle gemäß einer beispielhaften Ausführungsform der vorliegenden Erfindung in einem Längsschnitt;
- Fig. 2: eine erfindungsgemäße Kondensatfalle in einer zweiten beispielhaften Ausführungsform in einem Längsschnitt;

- Fig. 3: zeigt schematisch vereinfacht Abschnitte einer Single-Needle-Pneumatik-Einheit einer erfindungsgemäßen Blutbehandlungsvorrichtung; und
- Fig. 4: zeigt schematisch vereinfacht den Ablauf des erfindungsgemäßen Verfahrens in einer exemplarischen Ausführungsform.

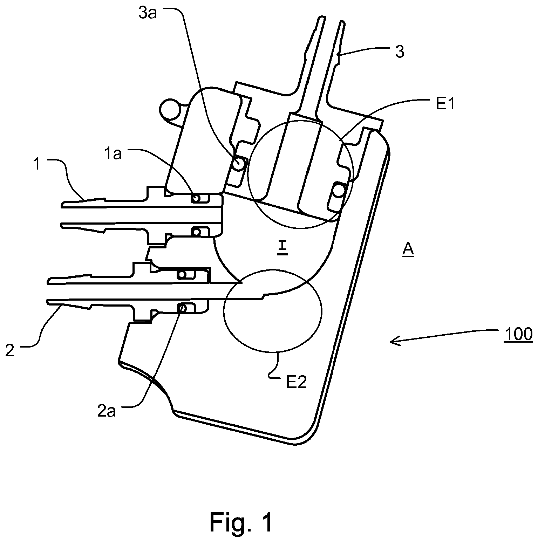

**Fig.** 1 zeigt eine Kondensatfalle 100 gemäß einer beispielhaften Ausführungsform der vorliegenden Erfindung in einem Längsschnitt.

Die Kondensatfalle 100 weist ein Inneres I auf, welches als Raum verstanden werden kann, der zur Aufnahme oder zum Durchleiten von Gas geeignet ist. Das Innere I kann hierzu, wie in Fig. 1 gezeigt, gegenüber einem Äußeren A der Kondensatfalle 100 abgedichtet sein, so dass ein Gasaustausch zwischen Innerem I und Äußerem A der Kondensatfalle 100 nur über Anschlüsse der Kondensatfalle möglich ist.

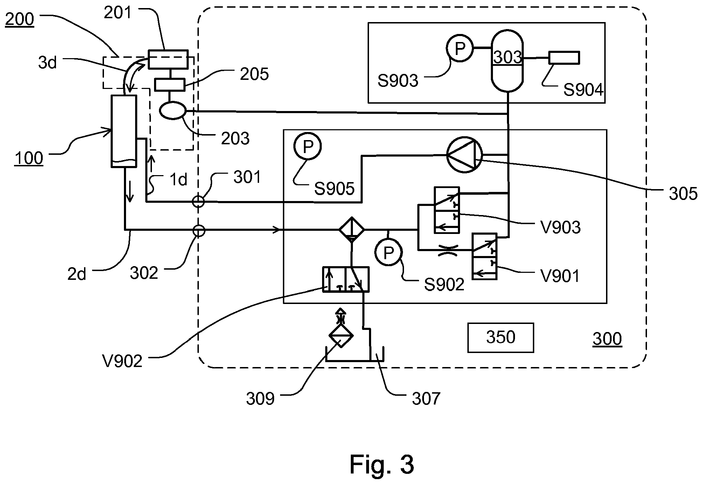

Bei den Anschlüssen, die die Kondensatfalle 100 hierzu aufweist, handelt es sich um einen ersten Anschluss 1 zur - beispielsweise direkten oder indirekten (also z. B. mittels Schlauchs) erfolgenden - Verbindung des Inneren I in Fluidkommunikation mit einem nur in Fig. 3 dargestellten Gasausgang 301 einer Blutbehandlungsvorrichtung 300, um einen zweiten Anschluss 2 zur Verbindung des Inneren I in Fluidkommunikation mit einem nur in Fig. 3 dargestellten Gaseingang 302 der Blutbehandlungsvorrichtung 300 und einen dritten Anschluss 3 zum Verbindung des Inneren I mit einem Gaseingang/Gasausgang, z. B. einem wiederum nur in Fig. 3 gezeigten Luftport oder Druckluftport 201 etwa eines Blutschlauchsatzes oder einer Blutkassette 200, siehe Fig. 3.

Eine Abdichtung zwischen Innerem I und Äußerem A kann mittels O-Ringe 1a, 2a und 3a oder auf andere Weise erfolgen.

Die Anschlüsse 1, 2 und/oder 3 können, wie in Fig. 1 exemplarisch gezeigt, als weibliche oder männliche Endstücke von Steckverbindern ausgestaltet sein. Sie können als Teile von Luer-Verbindern ausgestaltet sein. Sie können Bajonettverschluss-Elemente aufweisen. Sie können zur Verbindung mit Schläuchen vorgesehen oder verwendet werden.

Die Kondensatfalle 100 kann in jeder erfindungsgemäßen Ausführungsform optional aus POM (Polyoxymethylen) gefertigt sein oder POM aufweisen. Tüllen der Kondensatfalle 100 können passiviert sein, z. B. mittels eines Metalls oder Aluminiums, oder mittels dieser Materialien oder mit einem anderen Korrosionsschutz überzogen oder hiermit beschichtet sein.

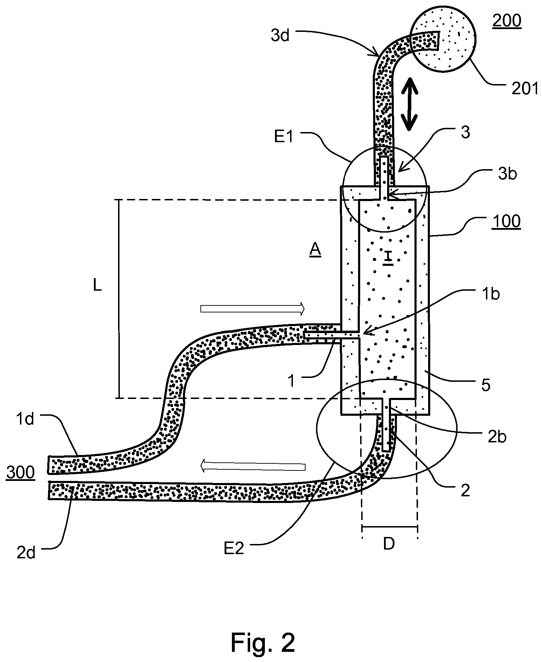

**Fig. 2** zeigt eine erfindungsgemäße Kondensatfalle 100 in einer zweiten beispielhaften Ausführungsform in einem Längsschnitt. Sein Inneres I hat eine Erstreckung D in Querrichtung und eine Erstreckung L in Längsrichtung. E1 und E2 bezeichnen in Fig. 2 den ersten bzw. zweiten Endbereich.

Das Innere I ist mittels eines Gehäuses 5 vom Äußeren A getrennt. Das Gehäuse 5 weist eine erste Öffnung 1b, eine zweite Öffnung 2b und eine dritte Öffnung 3b auf. Sie dienen dem Gasaustausch zwischen dem Inneren I und den mit der Kondensatfalle 100 verbundenen Gaseingängen und Gasausgängen 301, 302 und 201.

In der in Fig. 2 gezeigten, exemplarischen Ausführungsform ist der Abstand zwischen der ersten Öffnung 1b und der dritten Öffnung 3b mindestens so groß wie die Erstreckung D des Inneren I in dessen Querrichtung, beispielsweise also dessen Innendurchmesser. Der Innendurchmesser kann exemplarisch in einem Bereich von 1 mm bis 4 mm betragen, bevorzugt beträgt er zwischen 1,2 mm oder 2,0 mm, oder einen beliebigen Zwischenwert.

In der in Fig. 2 gezeigten exemplarischen Ausführungsform ist der Abstand zwischen der ersten Öffnung 1b und der zweiten Öffnung 2b optional mindestens so groß wie die Erstreckung D des Inneren I.

In Fig. 2 ist der erste Anschluss 1 mit einem Verbindungsschlauch 1d verbunden, welcher vorgesehen ist, das Innere I mit dem in Fig. 3 gezeigten Gasausgang 301 einer in Fig. 2 nur angedeuteten Blutbehandlungsvorrichtung 300 in Fluidverbindung zu verbinden.

In Fig. 2 ist der zweite Anschluss 2 mit einem Verbindungsschlauch 2d verbunden, welcher vorgesehen ist, das Innere I mit dem in Fig. 3 gezeigten Gaseingang 302 einer in Fig. 2 nur angedeuteten Blutbehandlungsvorrichtung 300 in Fluidverbindung zu verbinden.

In Fig. 2 ist der dritte Anschluss 3 mit einem Verbindungsschlauch 3d verbunden, welcher vorgesehen ist, das Innere I mit einem in Fig. 3 gezeigten Druckluftport oder Luftport 201 einer in Fig. 2 nur angedeuteten Blutkassette 200, in Fluidverbindung zu verbinden, wobei der Luftport 201 als Gaseingang und/oder Gasausgang der Blutkassette 200 dient. Der Verbindungsschlauch 3d ist möglichst kurz ausgestaltet, etwa 1 cm bis 6 cm, z.B. zwischen 2 cm und 5 cm, oder er weist beliebige Zwischenwerte auf.

Fig. 1 und Fig. 2 zeigen die Kondensatfalle 100 in einer Gebrauchstellung, also in einer senkrechten oder im Wesentlichen senkrechten Stellung, in welcher ein erster Endbereich E1 der Kondensatfalle 100 oder des Inneren I weiter oben als ein zweiter, diesem gegenüberliegender Endbereich E2 der Kondensatfalle 100 oder des Inneren I angeordnet ist.

Die neben Verbindungsleitungen 1d, 2d und 3d dargestellten Pfeile geben die Strömungsrichtung des Gases beim Betrieb der Kondensatfalle 100 an.

**Fig. 3** zeigt schematisch vereinfacht Abschnitte einer Single-Needle-Pneumatik-Einheit einer erfindungsgemäßen Blutbehandlungsvorrichtung 300. Obwohl die Fig. 3 dadurch einen Bezug der vorliegenden Erfindung zu einer Single-Needle-Behandlung herstellt, soll die vorliegende Erfindung hierauf nicht beschränkt werden. Die vorliegende Erfindung eignet sich vielmehr für ihren Einsatz bei jeder extrakorporalen Blutbehandlung, bei welcher mit Feuchtigkeit gesättigte Luft (oder allgemein: Gas) abwechselnd hin- und zurückgefördert wird, und bei welcher man sich Vorteile von der Entfernung von Feuchtigkeit oder Kondensat verspricht.

Die Single-Needle-Pneumatik der Fig. 3 weist optional einen Single-Needle-Tank 303 auf. Dieser dient dazu, Gas aus einer Single-Needle-Kammer 203 einer in Fig. 3 nur angedeuteten Blutbehandlungskassette 200 zwischenzuspeichern. Seine Kapazität kann rund 300 ml betragen.

Ein Drucksensor S903 und/oder ein Temperatursensor S904 können zum Messen von Druck bzw. Temperatur des im Single-Needle-Tank 303 gespeicherten Gases optional vorgesehen sein.

Weitere Drucksensoren S902 und S905 können stromauf oder stromab eines Kompressors 305 der Single-Needle-Pneumatik-Einheit vorgesehen sein, ferner können eine Bypassleitung und diese freischaltende Ventile V901 und/oder V903 vorgesehen sein.

Die in Fig. 3 gezeigte Single-Needle-Pneumatik-Einheit weist einen stromab des Kompressors 305 angeordneten Gasausgang 301 und einen stromauf des Kompressors 305 und/oder des Single-Needle-Tanks 303 angeordneten Gaseingang 302 auf. Sie sind in Fig. 3 mit den Verbindungsleitungen 1d bzw. 2d verbunden.

Wie Fig. 3 zu entnehmen ist, wird der Blutkassette 200 Zuluft zugeführt, nachdem diese - vom Kompressor 305 bewirkt - die Kondensatfalle 100 durchströmt. Die Zuluft wird in Fig. 3 direkt vom Kompressor 305 zugeführt. Dabei strömt die Zuluft über den im Gebrauchszustand der Kondensatfalle 100 höher gelegenen Abschluss 1 in das Innere I der Kondensatfalle 100 ein und von dort in Richtung zum Luftport 201 weiter.

Anders verhält es sich mit dem der Abluft des Luftports 201, also jener über den Luftport 201 aus der Blutkassetten 200 herausgeleiteten Luft. Diese strömt durch den - verglichen mit dem ersten Anschluss 1 - tiefer gelegenen zweiten Anschluss 2 in den Gaseingang 302 der Single-Needle-Pneumatik-Einheit. Möglichst direkt nach dem Gaseingang 302 wird der feuchte Anteil der Abluft aus dem Fluidstrom separiert und einem optionalen Kondensatauffang 307 zugeführt. Der Kondensatauffang 307 kann mit einer Einrichtung zum Verdunsten und /oder einer Belüftungs- oder Entlüftungseinrichtung 309 verbunden sein.

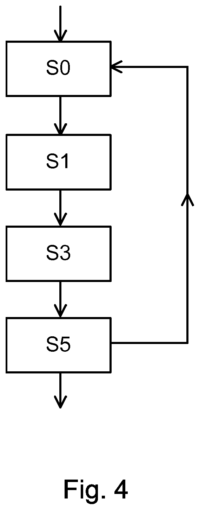

**Fig. 4** zeigt schematisch vereinfacht den Ablauf des erfindungsgemäßen Verfahrens in einer exemplarischen Ausführungsform.

Das Verfahren umfasst das Bereitstellen einer erfindungsgemäßen Blutbehandlungsvorrichtung, welche neben dem Kompressor 305 weiter eine Blutpumpe aufweist.

Die Blutbehandlungsvorrichtung 300 wird oder ist mit einer Blutkassette 200 verbunden, welche eine Single-Needle-Kammer 203 und einen Luftport 201 aufweist. Die Blutkassette 200 weist ferner eine Hydrophobmembran 205 auf, welche in einer Fluidverbindung zwischen der Single-Needle-Kammer 203 und dem Luftport 201 angeordnet ist.

Weiter weist die Blutbehandlungsvorrichtung 300 eine Steuervorrichtung 350 auf, welche in Fig. 3 schematisch gezeigt und welche programmiert ist, um wenigstens einmal einen Entlüftungsvorgang auszuführen oder zu bewirken, welcher folgende Schritte umfasst oder aus diesen besteht:
In einem optionalen Schritt S1 wird ein vorbestimmter Druck in der Single-Needle-Kammer 203 aufgebaut. Dies kann mittels der Blutpumpe und/oder mittels des Kompressors 305 erfolgen. Erfolgt der Druckaufbau mittels Kompressors, so geschieht dies vorzugsweise mit einer Förderrate, die nicht die Förderrate überschreitet, mit der in der venösen Phase, also beim Entleeren der Single-Needle-Kammer 203, Luft in die Single-Needle-Kammer 203 gefördert wird.

Im Schritt S3 wird der Druck in Richtung des Single-Needle-Tanks 303, oder in die Umgebung, abgelassen, um einen hohen Spitzenfluss zu erzielen. Dies kann schlagartig durch Öffnen eines Ventils erfolgen.

In einem wiederum optionalen Schritt S5 wird bei geöffnetem Ventil V903 zwischen Kondensatauffang und Kompressoreingang mittels Kompressor 305 gefördert. Dies dient dazu, das Kondensat in die Single-Needle-Pneumatik-Einheit zu fördern. Dort kann es über eine besondere Anordnung (z. B. eine Sammelkammer mit Entleerungsmöglichkeit am tiefsten Punkt der Single-Needle-Pneumatik-Einheit) abgeschieden werden.

Die Steuervorrichtung 350 kann programmiert sein, um den Entlüftungsvorgang mit den Schritten S1 bis S5 stets dann durchzuführen, wenn eine vorbestimmte Bedingung erfüllt ist. Zu den möglichen, vorbestimmten Bedingungen kann der Ablauf einer vorbestimmten Zeitdauer seit der zuletzt durchgeführten Entlüftung, das Erreichen/Unterschreiten einer vorbestimmten Temperatur, das Erreichen/Unterschreiten eines vorbestimmten Flussverhältnisses und/oder das Detektieren einer vorbestimmten Wellenlänge, z. B. bei Transmissions- oder Streulichtmessungen am Gaseingang 302, zählen. Das Vorliegen der vorbestimmten Bedingung kann in einem übergeordneten Schritt S0 überprüft werden.

### Bezugszeichenliste

- 100: Kondensatfalle
- 1: Erster Anschluss
- 1a: O-Ring
- 1b: Öffnung im Gehäuse
- 1d: Verbindungsleitung
- 2: Zweiter Anschluss
- 2a: O-Ring
- 2b: Öffnung im Gehäuse
- 2d: Verbindungsleitung
- 3: Dritter Anschluss
- 3a: O-Ring
- 3b: Öffnung im Gehäuse
- 3d: Verbindungsleitung
- 5: Gehäuse

- A: Äußeres
- D: Erstreckung in Querrichtung
- E1: Erster Endbereich
- E2: Zweiter Endbereich
- I: Inneres
- L: Erstreckung in Längsrichtung

- 200: Blutkassette
- 201: Luftport, Druckluftport
- 203: Single-Needle-Kammer
- 205: Hydrophobmembran; hydrophober Filter

- 300: Blutbehandlungsvorrichtung
- 301: Gasausgang
- 302: Gaseingang
- 303: Single-Needle-Tank
- 305: Kompressor
- 307: Kondensatauffang
- 309: Belüftungs- oder Entlüftungseinrichtung
- 350: Steuervorrichtung

- S902: Sensor
- S903: Sensor
- S904: Sensor
- S905: Sensor
- V901: Ventil
- V903: Ventil

## Patentansprüche

1. Medizinische Kondensatfalle (100) zur Verwendung bei einer mittels einer Blutbehandlungsvorrichtung (300) erfolgenden externen Blutbehandlung, wobei die Kondensatfalle (100) aufweist:
- wenigstens ein Inneres (I);
- wenigstens einen ersten Anschluss (1) zur Verbindung des Inneren (I) in Fluidkommunikation mit einem Gasausgang (301) der Blutbehandlungsvorrichtung (300);
- wenigstens einen zweiten Anschluss (2) zur Verbindung des Inneren (I) in Fluidkommunikation mit einem Gaseingang (302) der Blutbehandlungsvorrichtung (300); und
- wenigstens einen dritten Anschluss (3) zur Verbindung des Inneren (I) mit einem Luftport (201) einer Blutbehandlungseinrichtung, etwa einem Blutschlauchsatz oder einer Blutkassette (200), **dadurch gekennzeichnet, dass** der dritte Anschluss (3) in einer Gebrauchsstellung der Kondensatfalle (100) höher angeordnet ist als der erste Anschluss (1) und der zweite Anschluss (2), und wobei der erste Anschluss (1) in einer Gebrauchsstellung der Kondensatfalle (100) höher angeordnet ist als der zweite Anschluss (2).

2. Medizinische Kondensatfalle (100) nach Anspruch 1, wobei der dritte Anschluss (3) einem ersten Endbereich (E1) des Inneren (I) oder der Kondensatfalle (100) zugeordnet ist, wobei der zweite Anschluss (2) einem zweiten, dem ersten Endbereich (E1) gegenüberliegenden zweiten Endbereich (E2) des Inneren (I) oder der Kondensatfalle (100) zugeordnet ist, und wobei der erste Anschluss (1) zwischen dem zweiten Anschluss (2) und dem dritten Anschluss (3) angeordnet ist.

3. Medizinische Kondensatfalle (100) nach einem der vorangegangenen Ansprüche,
- wobei das Innere (I) mittels eines Gehäuses (5) vom Äußeren (A) der Kondensatfalle (100) getrennt ist, wobei das Innere (I) eine Erstreckung (L) in Längsrichtung und eine Erstreckung (D) in Querrichtung hiervon hat; und
- wobei das Gehäuse (5) wenigstens eine erste Öffnung (1b) für einen Gasaustausch mit dem Inneren (I) über den ersten Anschluss (1), wenigstens eine zweite Öffnung (2b) für einen Gasaustausch mit dem Inneren (I) über den zweiten Anschluss (2) und wenigstens eine dritte Öffnung (3b) für einen Gasaustausch mit dem Inneren (I) über den dritten Anschluss (3) aufweist.

4. Medizinische Kondensatfalle (100) nach Anspruch 3, wobei der Abstand zwischen der ersten Öffnung (1b) und der dritten Öffnung (3b) mindestens der Erstreckung (D) in Querrichtung entspricht.

5. Medizinische Kondensatfalle (100) nach einem der Ansprüche 3 oder 4, wobei der Abstand zwischen der ersten Öffnung (1b) und der zweiten Öffnung (2b) mindestens der Erstreckung (D) in Querrichtung entspricht.

6. Medizinische Kondensatfalle (100) nach einem der Ansprüche 3 bis 5, wobei ein Durchmesser und/oder eine Fläche oder eine Länge einer Fläche der ersten Öffnung (1b), der zweiten Öffnung (2b) und/oder der dritten Öffnung (3b) oder die Innendurchmesser der mit den Anschlüssen (1, 2, 3) verbundenen Schläuche oder Verbindungsleitungen (1d, 2d, 3d) kleiner ist als die Erstreckung (D) des Inneren (I) in Querrichtung.

7. Verfahren zum Entfeuchten wenigstens eines Abschnitts einer Blutbehandlungsvorrichtung (300) oder einer Blutbehandlungseinrichtung (200), welches die folgenden Schritte umfasst oder hieraus besteht:
- Bereitstellen einer Blutbehandlungsvorrichtung (300), einer Kondensatfalle (100) gemäß einem der vorangegangenen Ansprüche und einer Blutbehandlungseinrichtung (200), wobei:
- die Blutbehandlungsvorrichtung (300) einen Gasausgang (301) und einen getrennt hiervon ausgestalteten Gaseingang (302) aufweist, wobei sie weiter eine Blutpumpe aufweist, und wobei sie ferner einen Kompressor (305) zum Erzeugen von Druckluft aufweist, welcher in Fluidverbindung mit dem Gasausgang (301) steht;
- die Blutbehandlungseinrichtung eine Blutleitung oder Blutkammer, einen Luftport (201) und eine Hydrophobmembran (205) aufweist, wobei der Luftport (201) in Fluidkommunikation mit der Hydrophobmembran (205) auf einer luftport-zugewandten Seite der Hydrophobmembran (205) angeordnet ist; wobei
- der Luftport (201) der Blutbehandlungseinrichtung mit dem Gasausgang (301) der Blutbehandlungsvorrichtung (300) verbunden ist; und wobei die Blutleitung oder die Blutkammer mit der Blutpumpe, insbesondere ebenfalls in Fluidkommunikation mit dem Luftport (201), verbunden ist;
wobei die Kondensatfalle (100) aufweist:
- wenigstens ein Inneres (I);
- wenigstens einen ersten Anschluss (1) zur Verbindung des Inneren (I) in Fluidkommunikation mit dem Gasausgang (301) der Blutbehandlungsvorrichtung (300), verbunden mit dem Gasausgang (301);
- wenigstens einen zweiten Anschluss (2) zur Verbindung des Inneren (I) in Fluidkommunikation mit einem Gaseingang (302) der Blutbehandlungsvorrichtung (300), verbunden mit dem Gaseingang (302); und
- wenigstens einen dritten Anschluss (3) zur Verbindung des Inneren (I) mit einem Luftport (201) der Blutbehandlungseinrichtung (300), verbunden mit dem Luftport (201),
- wenigstens einmaliges Durchführen eines Entlüftungsvorgangs mit den folgenden Schritten:
- Aufbauen eines vorbestimmten Drucks oder Mindestdrucks innerhalb der Blutbehandlungseinrichtung (300) auf der luftport-abgewandten Seite der Hydrophobmembran (205) in Richtung des ersten Anschlusses (1) mittels der Blutpumpe und/oder mittels des Kompressors (305); und
- Abbau des Drucks in Richtung der
Hydrophobmembran (205).

8. Verfahren nach Anspruch 7, wobei
- die Blutbehandlungsvorrichtung (300) einen Single-Needle-Tank (303) aufweist,
- die Blutbehandlungseinrichtung eine Blutkassette (200) ist,
- die Blutleitung oder Blutkammer eine Single-Needle-Kammer (203) ist oder umfasst,
- die Hydrophobmembran (205) in einer Fluidverbindung zwischen dem Luftport (201) und der Single-Needle-Kammer (203) angeordnet ist,
- der vorbestimmte Druck oder Mindestdruck in der Single-Needle-Kammer (203) aufgebaut wird,
- der Druck in Richtung eines Single-Needle-Tanks (303) der Blutbehandlungsvorrichtung (300) oder in die Umgebung abgebaut wird, und wobei
- der Abschnitt der Blutbehandlungsvorrichtung (300), in den optional mittels Kompressors (305) Kondensat gefördert wird, die Single-Needle-Pneumatik-Einheit der Blutbehandlungsvorrichtung (300) ist.

9. Verfahren nach einem der Ansprüche 7 bis 8, wobei das Verfahren weiter umfasst:
- Durchführen des Entlüftungsvorgangs, wenn eine der folgenden Bedingungen erfüllt ist:
- Ablauf einer vorbestimmten Zeitdauer seit der zuletzt durchgeführten Entlüftung;
- bei Erreichen/Unterschreiten einer vorbestimmten Temperatur, gemessen mit einem Temperatursensor der Blutbehandlungsvorrichtung (300);
- bei Erreichen/Unterschreiten eines vorbestimmten Flussverhältnisses, gemessen mit einem Flusssensor der Blutbehandlungsvorrichtung (300);
- bei Detektieren einer vorbestimmten Wellenlänge bei Transmissions- oder Streulichtmessungen am Gaseingang (302).

10. Blutbehandlungsvorrichtung (300), aufweisend wenigstens eine Einrichtung zum Abscheiden von Kondensat, ausgestaltet als Kondensatfalle (100), oder hiermit verbunden, wobei die Blutbehandlungsvorrichtung (300) einen Gasausgang (301) und einen getrennt hiervon ausgestalteten Gaseingang (302) aufweist, wobei die Blutbehandlungsvorrichtung (300) einen Kompressor (305) und einen Kondensatauffang (307) aufweist, wobei der Kompressor (305) mit dem Gasausgang (301) verbunden ist, und wobei der Kondensatauffang (307) mit dem Gaseingang (302) verbunden ist,
wobei die Kondensatfalle (100) aufweist:
- wenigstens ein Inneres (I);
- wenigstens einen ersten Anschluss (1) zur Verbindung des Inneren (I) in Fluidkommunikation mit dem Gasausgang (301) der Blutbehandlungsvorrichtung (300), verbunden mit dem Gasausgang (301);
- wenigstens einen zweiten Anschluss (2) zur Verbindung des Inneren (I) in Fluidkommunikation mit einem Gaseingang (302) der Blutbehandlungsvorrichtung (300), verbunden mit dem Gaseingang (302); und
- wenigstens einen dritten Anschluss (3) zur Verbindung des Inneren (I) mit einem Luftport (201) der Blutbehandlungseinrichtung (300), verbunden mit dem Luftport (201).

11. Blutbehandlungsvorrichtung (300) nach Anspruch 10, wobei die Blutbehandlungsvorrichtung (300) verbunden ist mit einer Blutbehandlungseinrichtung, ausgestaltet als Blutschlauchsatz und/oder Blutkassette (200), welche eine Hydrophobmembran (205) aufweist, wobei die Kondensatfalle (100) in einer Fluidverbindung zwischen der Hydrophobmembran (205) und dem Gasausgang (301) und/oder Gaseingang (303) angeordnet ist.

12. Blutbehandlungsvorrichtung nach einem der Ansprüche 10 bis 11, ausgestaltet als Akut-Dialysevorrichtung, Dialysevorrichtung, Hämodialysevorrichtung, Hämofiltrationsvorrichtung, Apheresevorrichtung, Plasmabehandlungs- oder Austauschvorrichtung, TPE-Vorrichtung, und/oder Kombinationen hiervon.

13. Blutbehandlungsvorrichtung (300) nach einem der Ansprüche 10 bis 12, mit einer Steuervorrichtung (350), programmiert, um Schritte des Verfahrens gemäß einem der Ansprüche 7 bis 9 auszuführen oder zu bewirken.

14. Blutbehandlungseinrichtung, ausgestaltet als Blutschlauchsatz und/oder Blutkassette (200), welche eine Kondensatfalle (100) gemäß einem der Ansprüche 1 bis 6 umfasst oder hiermit verbunden ist.

## Claims

1. A medical condensate trap (100) for use in an external blood treatment carried out by means of a blood treatment apparatus (300), the condensate trap (100) comprising:
- at least one interior (I);
- at least one first connector (1) for connecting the interior (I) in fluid communication with a gas outlet (301) of the blood treatment apparatus (300);
- at least one second connector (2) for connecting the interior (I) in fluid communication with a gas inlet (302) of the blood treatment apparatus (300); and
- at least one third connector (3) for connecting the interior (I) with an air port (201) of a blood treatment device, such as a blood tubing set or a blood cassette (200),
**characterized in that**
in a use position of the condensate trap (100),
the third connector (3) is arranged higher than the first connector (1) and the second connector (2),
and wherein in a position of use of the condensate trap (100) , the first connector (1) is arranged higher than the second connector (2).

2. The medical condensate trap (100) according to claim 1,
wherein the third connector (3) is assigned to a first end portion (E1) of the interior (I) or of the condensate trap (100), wherein the second connector (2) is assigned to a second end portion (E2) of the interior (I) or of the condensate trap (100) facing the first end portion (E1), and wherein the first connector (1) is arranged between the second connector (2) and the third connector (3).

3. The medical condensate trap (100) according to anyone of the preceding claims,
- wherein the interior (I) is separated from the exterior (A) of the condensate trap (100) by means of a housing (5), the interior (I) having an extension (L) in a longitudinal direction and an extension (D) in a transversal direction thereof; and
- wherein the housing (5) comprises at least one first opening (1b) for a gas exchange with the interior (I) via the first connector (1), at least one second opening (2b) for a gas exchange with the interior (I) via the second connector (2) and at least one third opening (3b) for a gas exchange with the interior (I) via the third connector (3) .

4. The medical condensate trap (100) according to claim 3,
wherein the distance between the first opening (1b) and the third opening (3b) corresponds at least to the extension (D) in the transversal direction.

5. The medical condensate trap (100) according to anyone of claims 3 or 4, wherein the distance between the first opening (1b) and the second opening (2b) corresponds at least to the extension (D) in the transversal direction.

6. The medical condensate trap (100) according to anyone of claims 3 to 5, wherein a diameter and/or a surface or a length of a surface of the first opening (1b), of the second opening (2b) and/or of the third opening (3b) or inner diameters of hoses or of connecting lines (1d, 2d, 3d) connected to the connectors (1, 2, 3) are smaller than the extension (D) of the interior (I) in the transversal direction.

7. A method for dehumidifying at least one section of a blood treatment apparatus (300) or of a blood treatment device (200), which comprises or consists of the following steps:
- providing a blood treatment apparatus (300), a condensate trap (100) according to anyone of the preceding claims and a blood treatment device (200), wherein
- the blood treatment apparatus (300) comprises a gas outlet (301) and a gas inlet (302) designed separately therefrom, wherein it further comprises a blood pump, and wherein it also comprises a compressor (305) for generating compressed air being in fluid communication with the gas outlet (301);
- the blood treatment device comprises a blood line or a blood chamber, an air port (201) and a hydrophobic membrane (205), the air port (201) being arranged in fluid communication with the hydrophobic membrane (205) on a side of the hydrophobic membrane (205) facing the air port; wherein
- the air port (201) of the blood treatment device is connected to the gas outlet (301) of the blood treatment apparatus (300) ; and wherein the blood line or the blood chamber is connected to the blood pump, which is notably also in fluid communication with the air port (201);
wherein the condensate trap (100) comprises:
- at least one interior (I);
- at least one first connector (1) connected to the gas outlet (301), for connecting the interior (I) in fluid communication with the gas outlet (301) of the blood treatment apparatus (300);
- at least one second connector (2) connected to the gas inlet (302), for connecting the interior (I) in fluid communication with a gas inlet (302) of the blood treatment apparatus (300); and
- at least one third connector (3) connected to the air port (201), for connecting the interior (I) with an air port (201) of the blood treatment device,
- performing a venting process at least once according to the following steps:
- building up a predetermined pressure or minimum pressure within the blood treatment device by means of the blood pump and/or by means of the compressor (305) , on the side of the hydrophobic membrane (205) facing the air port, in the direction of the first connector (1); and
- reducing the pressure in the direction of the hydrophobic membrane (205).

8. The method according to claim 7, wherein
- the blood treatment apparatus (300) comprises a single-needle tank (303),
- the blood treatment device is a blood cassette (200),
- the blood line or the blood chamber is, or comprises, a single-needle chamber (203),
- the hydrophobic membrane (205) is arranged in a fluid communication between the air port (201) and the single-needle chamber (203),
- the predetermined pressure or minimum pressure is built up in the single-needle chamber (203),
- the pressure in the direction of a single-needle tank (303) of the blood treatment apparatus (300) or in the environment is reduced, and wherein
- the section of the blood treatment apparatus (300) into which condensate is optionally conveyed by means of a compressor (305), is the single-needle pneumatic unit of the blood treatment apparatus (300).

9. The method according to anyone of claims 7 to 8, wherein the method further comprises:
- performing the venting process, if one of the following conditions is met:
- elapse of a predetermined period of time since the last venting was performed;
- achieving or falling below a predetermined temperature measured with a temperature sensor of the blood treatment apparatus (300);
- achieving or falling below a predetermined flow ratio measured with a flow sensor of the blood treatment apparatus (300);
- detecting a predetermined wavelength during transmission or scattering light measurements at the gas inlet (302) .

10. A blood treatment apparatus (300) comprising at least a device for separating condensate, designed as a condensate trap (100), or connected thereto, wherein the blood treatment apparatus (300) comprises a gas outlet (301) and a gas inlet (302) separately designed therefrom,
the blood treatment apparatus (300) comprising a compressor (305) and a condensate collector (307), the compressor (305) being connected to the gas outlet (301), and the condensate collector (307) being connected to the gas inlet (302),
wherein the condensate trap (100) comprises:
- at least one interior (I);
- at least one first connector (1) connected with the gas outlet (301), for connecting the interior (I) in fluid communication with the gas outlet (301) of the blood treatment apparatus (300);
- at least one second connector (2) connected with the gas inlet (302), for connecting the interior (I) in fluid communication with a gas inlet (302) of the blood treatment apparatus (300); and
- at least one third connector (3) connected with an air port (201), for connecting the interior (I) with the air port (201) of the blood treatment device (200).

11. The blood treatment apparatus (300) according to claim 10, wherein the blood treatment apparatus (300) is connected with a blood treatment device, designed as a blood tubing set and/or as a blood cassette (200), which comprises a hydrophobic membrane (205), wherein the condensate trap (100) is arranged in a fluid communication between the hydrophobic membrane (205) and the gas outlet (301) and/or the gas inlet (302).

12. The blood treatment apparatus according to anyone of claims 10 to 11, designed as an acute dialysis apparatus, dialysis apparatus, hemodialysis apparatus, hemofiltration apparatus, apheresis apparatus, plasma treatment or exchange apparatus, TPE apparatus, and/or combinations thereof.

13. The blood treatment apparatus (300) according to anyone of claims 10 to 12, comprising a control apparatus (350) programmed to perform or effect the steps of the method according to anyone of claims 7 to 9.

14. A blood treatment device designed as a blood tubing set and/or as a blood cassette (200), which comprises or is connected with a condensate trap (100) according to anyone of claims 1 to 6.

## Revendications

1. Un piège à condensat médical (100) médical destiné à être utilisé dans un traitement sanguin externe réalisé au moyen d'un appareil de traitement du sang (300), le piège à condensat (100) comprenant:
- au moins un intérieur (I);
- au moins un premier raccord (1) pour raccorder l'intérieur (I) en communication fluidique avec une sortie de gaz (301) de l'appareil de traitement du sang (300);
- au moins un second raccord (2) pour raccorder l'intérieur (I) en communication fluidique avec une entrée de gaz (302) de l'appareil de traitement du sang (300) ; et
- au moins un troisième raccord (3) pour raccorder l'intérieur (I) à un port d'aération (201) d'un dispositif de traitement du sang, tel qu'un set de tuyaux sanguins ou une cassette à sang (200),
**caractérisé en ce que**
en position d'utilisation du piège à condensat (100),
le troisième raccord (3) est agencé plus haut que le premier raccord (1) et que le second raccord (2),
et **en ce que**, en position d'utilisation du piège à condensat (100), le premier raccord (1) est agencé plus haut que le second raccord (2).

2. Le piège à condensat médical (100) selon la première revendication, où le troisième raccord (3) est affecté à une première partie terminale (E1) de l'intérieur (I) ou du piège à condensat (100), où le second raccord (2) est affecté à une seconde partie terminale (E2) de l'intérieur (I) ou du piège à condensat (100) opposée à la première partie terminale (E1) , et où le premier raccord (1) est agencé entre le second raccord (2) et le troisième raccord (3).

3. Le piège à condensat médical (100) selon l'une quelconque des revendications précédentes,
- où l'intérieur (I) est séparé de l'extérieur (A) du piège à condensat (100) au moyen d'un boîtier (5), l'intérieur (I) ayant une extension (L) dans une direction longitudinale ainsi qu'une extension (D) dans une direction transversale de celui-ci; et
- où le boîtier (5) comprend au moins une première ouverture (1b) pour un échange gazeux avec l'intérieur (I) via le premier raccord (1), au moins une seconde ouverture (2b) pour un échange gazeux avec l'intérieur (I) via le second raccord (2) et au moins une troisième ouverture (3b) pour un échange gazeux avec l'intérieur (I) via le troisième raccord (3).

4. Le piège à condensat médical (100) selon la revendication 3, où la distance entre la première ouverture (1b) et la troisième ouverture (3b) correspond au moins à l'extension (D) dans la direction transversale.

5. Le piège à condensat médical (100) selon l'une quelconque des revendications 3 ou 4, où la distance entre la première ouverture (1b) et la seconde ouverture (2b) correspond au moins à l'extension (D) dans la direction transversale.

6. Le piège à condensat médical (100) selon l'une quelconque des revendications 3 à 5, où un diamètre et/ou une surface ou une longueur d'une surface de la première ouverture (1b), de la seconde ouverture (2b) et/ou de la troisième ouverture (3b) ou les diamètres intérieurs des tuyaux ou des conduites de raccordement (1d, 2d, 3d) reliées aux raccords (1, 2, 3) sont inférieurs à l'extension (D) de l'intérieur (I) dans la direction transversale.

7. Un procédé de déshumidification d'au moins une partie d'un appareil de traitement du sang (300) ou d'un dispositif de traitement du sang (200), qui comprend ou consiste en les étapes suivantes:
- la mise à disposition d'un appareil de traitement du sang (300), d'un piège à condensat (100) selon l'une quelconque des revendications précédentes, ainsi que d'un dispositif de traitement du sang (200), où:
- l'appareil de traitement du sang (300) comprend une sortie de gaz (301) ainsi qu'une arrivée de gaz (302) configurée séparément de cette dernière, où il comprend en outre une pompe à sang et où il comprend également un compresseur (305) pour générer de l'air comprimé, celui-ci étant en communication fluidique avec la sortie de gaz (301);
- le dispositif de traitement du sang comprend une conduite de sang ou une chambre à sang, un port d'aération (201) ainsi qu'une membrane hydrophobe (205), le port d'aération (201) étant agencé en communication fluidique avec la membrane hydrophobe (205) sur un côté de la membrane hydrophobe (205) faisant face au port d'aération; où
- le port d'aération (201) du dispositif de traitement du sang est relié à la sortie de gaz (301) de l'appareil de traitement du sang (300); et où la conduite de sang ou la chambre à sang est reliée à la pompe à sang, qui est notamment également en communication fluidique avec le port d'aération (201);
où le piège à condensat (100) comprend:
- au moins un intérieur (I);
- au moins un premier raccord (1), raccordé à la sortie de gaz (301), pour raccorder l'intérieur (I) en communication fluidique avec la sortie de gaz (301) de l'appareil de traitement du sang (300);
- au moins un second raccord (2), raccordé à l'arrivée de gaz (302), pour raccorder l'intérieur (I) en communication fluidique avec l'arrivée de gaz (302) de l'appareil de traitement du sang (300); et
- au moins un troisième raccord (3), raccordé au port d'aération (201), pour raccorder l'intérieur (I) avec un port d'aération (201) du dispositif de traitement du sang,
- l'exécution, au moins une fois, d'un processus de ventilation selon les étapes suivantes:
- l'établissement d'une pression prédéterminée ou d'une pression minimale, au moyen de la pompe à sang et/ou au moyen du compresseur (305), à l'intérieur du dispositif de traitement du sang, du côté de la membrane hydrophobe (205) opposé au port d'aération, vers le premier raccord (1); et
- la réduction de la pression vers la membrane hydrophobe (205).

8. Le procédé selon la revendication 7, où
- l'appareil de traitement du sang (300) comprend un réservoir single-needle (303),
- le dispositif de traitement du sang est une cassette à sang (200),
- la conduite de sang ou la chambre à sang, est ou comprend, une chambre single-needle (203),
- la membrane hydrophobe (205) est agencée en communication fluidique entre le port d'aération (201) et la chambre single-needle (203),
- la pression prédéterminée ou pression minimale est établie dans la chambre single-needle (203),
- la pression vers un réservoir single-needle (303) de l'appareil de traitement du sang (300) ou dans l'environnement est réduite, et où
- la partie de l'appareil de traitement du sang (300), dans laquelle le condensat est acheminé éventuellement au moyen d'un compresseur (305), est l'unité pneumatique single-needle de l'appareil de traitement du sang (300).

9. Le procédé selon l'une quelconque des revendications 7 à 8, où le procédé comprend en outre :
- l'exécution d'un processus de ventilation, si l'une des conditions suivantes est remplie:
- l'écoulement d'un laps de temps prédéterminé depuis la dernière ventilation effectuée;
- l'atteinte ou le sous-dépassement d'une température prédéterminée, mesurée à l'aide d'un capteur de température de l'appareil de traitement du sang (300);
- l'atteinte ou le sous-dépassement d'un débit prédéterminé, mesuré à l'aide d'un capteur de débit de l'appareil de traitement du sang (300);
- la détection d'une longueur d'onde prédéterminée lors de mesures de transmission ou de lumière diffusée à l' arrivée de gaz (302).

10. Un appareil de traitement du sang (300) comprenant au moins un dispositif de séparation de condensat, conçu comme piège à condensat (100), ou relié à celui-ci, où l'appareil de traitement du sang (300) comprend une sortie de gaz (301) et une arrivée de gaz (302) conçue séparément de celle-ci, l'appareil de traitement du sang (300) comprenant un compresseur (305) ainsi qu'un collecteur de condensat (307), le compresseur (305) étant relié à la sortie de gaz (301), et le collecteur de condensat (307) étant relié à l'arrivée de gaz (302),
où le piège à condensat (100) comprend:
- au moins un intérieur (I);
- au moins un premier raccord (1), relié à la sortie de gaz (301), pour raccorder l' intérieur (I) en communication fluidique avec la sortie de gaz (301) de l'appareil de traitement du sang (300);
- au moins un second raccord (2), relié à l'arrivée de gaz (302), pour raccorder l'intérieur (I) en communication fluidique avec une arrivée de gaz (302) de l'appareil de traitement du sang (300); et
- au moins un troisième raccord (3), relié au port d'aération (201), pour raccorder l'intérieur (I) à un port d'aération (201) du dispositif de traitement du sang (200).

11. L'appareil de traitement du sang (300) selon la revendication 10, où l'appareil de traitement du sang (300) est relié à un dispositif de traitement du sang, conçu comme un set de tuyaux sanguins et/ou une cassette à sang (200) qui comporte une membrane hydrophobe (205), où le piège à condensat (100) est agencé en communication fluidique entre la membrane hydrophobe (205) et la sortie de gaz (301) et/ou l'arrivée de gaz (302).

12. L'appareil de traitement du sang selon l'une quelconque des revendications 10 à 11, conçu comme un appareil de dialyse aiguë, un appareil de dialyse, un appareil d'hémodialyse, un appareil d'hémofiltration, un appareil d'aphérèse, un appareil de traitement ou d'échange de plasma, un appareil TPE, et/ou des combinaisons de ceux-ci.

13. L'appareil de traitement du sang (300) selon l'une quelconque des revendications 10 à 12, comprenant un appareil de commande (350) programmé pour exécuter ou effectuer des étapes du procédé selon l'une quelconque des revendications 7 à 9.

14. Un dispositif de traitement du sang, conçu comme un set de tuyaux sanguins et/ou une cassette à sang (200), qui comprend ou est relié à un piège à condensat (100) selon l'une quelconque des revendications 1 à 6.
